# EUROPÄISCHE PATENTSCHRIFT

(11) **EP 1 276 476 B1**
(45) Veröffentlichungstag und Bekanntmachung des Hinweises auf die Patenterteilung: **21.06.2006**
(21) Anmeldenummer: 01949281.8
(22) Anmeldetag: 07.04.2001
(51) Int. Cl.: A61K 31/00, A61K 31/4168, A61K 31/55, A61K 45/06, A61P 9/00

(54) **VERWENDUNG VON BRADYCARDICA BEI DER BEHANDLUNG VON MIT HYPERTROPHIE EINHERGEHENDEN MYOCARDERKRANKUNGEN UND NEUE ARZNEIMITTELKOMBINATIONEN**
USE OF BRADYCARDIAC SUBSTANCES IN THE TREATMENT OF MYOCARDIAL DISEASES ASSOCIATED WITH HYPERTROPHY AND NOVEL MEDICAMENT COMBINATIONS
UTILISATION D'AGENTS BRADYCARDIQUES DANS LE TRAITEMENT DE MALADIES MYOCARDIAQUES ACCOMPAGNEES D'HYPERTROPHIE ET NOUVELLES COMBINAISONS PHARMACEUTIQUES

(30) Priorität: 13.04.2000 DE 10018401
(43) Veröffentlichungstag der Anmeldung: 22.01.2003
(60) Teilanmeldung: 06111485.6
(73) Patentinhaber: Boehringer Ingelheim Pharma GmbH & Co.KG, 55218 Ingelheim am Rhein (DE)
(72) Erfinder: DÄMMGEN, Jürgen, 88416 Ochsenhausen (DE); GUTH, Brian, 88447 Warthausen (DE); SEIDLER, Randolph, 88441 Mittelbiberach (DE)
(86) Internationale Anmeldenummer: PCT/EP2001/004034
(87) Internationale Veröffentlichungsnummer: WO 2001/078699

(56) Entgegenhaltungen:
- EP-A- 0 471 388
- WO-A-00/02543
- US-A- 5 595 987
- GREGOR, P. ET AL: "Use of Verapamil in the treatment of hypertrophic cadiomyopathy" COR ET VASA, Bd. 28, Nr. 6, 1986, Seiten 404-412, XP001024692
- HARTMANN A. ET AL: "Persisting effect of Calcium-channel blockers on left ventricular function in hypertrophic cardiomyopathy after 14 years' treatment" ANGIOLOGY, Bd. 47, Nr. 8, 1996, Seiten 765-773, XP001024817
- TOSHIMA H. ET AL: "Comparable effects of oral diltiazem and verapamil in the treatment of hypertrophic cardiomyopathy" JAPANESE HEART JOURNAL, Bd. 27, Nr. 5, 1986, Seiten 701-715, XP001024687
- KOBER G. ET AL: "Clinical cardiology: hypertrophic cardiomyopathy. Long-term verapamil versus propranolol treatment of hypertrophic cardiomyopathy in matched pairs of patients" CIRCULATION SUPPLEMENT, ABSTRACTS FROM THE 60TH SCIENTIFIC SESSION, Bd. 76, Nr. 4, 1987, Seite IV-248 XP001024696
- EPSTEIN S.E. ET AL: "Verapamil: its potential for causing serious complications in patients with hypertrophic cardiomyopathy" CIRCULATION, Bd. 64, Nr. 3, 1981, XP001024694
- DATABASE WPI Section PQ, Week 199747 Derwent Publications Ltd., London, GB; Class P31, AN 1997-510672 XP002179143 & RU 2 078 536 C (UKR CARDIOLOGY RES INST) , 10. Mai 1997 (1997-05-10)
- "The Extra Pharmacopoeia, 30th Edition" , THE PHARMACEUTICAL PRESS , LONDON XP002179142 Seite 668, linke Spalte Absatz mit dem Titel: Calcium-channel blockers
- SHINKE T (REPRINT) ET AL: "Beneficial effects of heart rate reduction on cardiac mechanics and energetics in patients with left ventricular dysfunction" JAPANESE CIRCULATION JOURNAL-ENGLISH EDITION, (DEC 1999) VOL. 63, NO. 12, PP. 957-964. PUBLISHER: BLACKWELL SCIENCE ASIA, 54 UNIVERSITY ST, P O BOX 378, CARLTON VICTORIA 3053, AUSTRALIA. ISSN: 0047-1828., XP001041863 KOBE UNIV, SCH MED, DEPT INTERNAL MED 1, CHUO KU, 7-5-2 KUSUNOKI CHO, KOBE, HYOGO 6500014, JAPAN (Reprint)
- RIEU, J. P. (1) ET AL: "Synthesis and bradycardia activity of a series of substituted 3-aminoalkyl-2,3-dihydro-4H-1,3-benzoxazin -4-ones as potent antiischemics" EUROPEAN JOURNAL OF MEDICINAL CHEMISTRY, (1993) VOL. 28, NO. 9, PP. 683-691. , XP001041845
- NICHOLLS D P ET AL: "CARDIO VASCULAR EFFECTS OF ALINIDINE AND PROPRANOLOL ALONE AND IN COMBINATION WITH HYDRALAZINE IN NORMAL MAN." BR J CLIN PHARMACOL, (1983) 15 (1), 21-30, XP001041903

## Beschreibung

Zur Behandlung einer erhöhten Herzfrequenz können bradykarde Substanzen eingesetzt werden, insbesondere Ca⁺⁺ Kanal Blocker wie Diltiazem und Verapamil oder Beta-Rezeptorenblocker wie A-tenolol, Bisoprolol, Carvedolol, Metoprolol oder Propanolol und i_{f}-Kanalblocker wie Zatebradine [1-(7,8-Dimethoxy-1,3,4,5-tetrahydro-2H-3-benzazepin-2-on-3-yl)-3-[N-methyl-N-(2-(3,4-dimethoxy-phenyl)-ethyl)-propan] (siehe EP-B-0 065 229), 3-[(N-(2-(3,4-Dimethoxy-phenyl)-ethyl)-piperidin-3-yl)-methyl]-(7,8-dimethoxy-1,3,4,5-tetrahydro-2H-3-benzazepin-2-on (siehe EP-B-0 224 794) und dessen Enantiomer Cilobradine [(+)-3-[(N-(2-(3,4-Dimethoxy-phenyl)-ethyl)-piperidin-3-(S)-yl)-methyl]-(7,8-dimethoxy-1,3,4,5-tetrahydro-2H-3-benzazepin-2-on] oder Alinidine [2-(N-Allyl-2,6-dichlor-anilino)-2-imidazolidin), siehe auch US-Patent Nr. 3,708,485], wobei von Zatebradine auch bekannt ist, dass es eine günstige Wirkung bei der Behandlung der Herzinsuffizienz (siehe EP-B-0 471 388) aufweist.

Ferner ist bekannt, dass bradykarde Substanzen, insbesondere die vorstehend erwähnten Verbindungen, wobei die i_{f}-Kanalblocker wie Zatebradine, Cilobradine oder Alinidine insbesondere jedoch Cilobradine bevorzugt sind, die Symptomatik von mit Hypertrophie einhergehenden Myocarderkrankungen, insbesondere zur Behandlung von idiopathischen hypertrophischen Kardiomyopathien (HCM) wie Hypertrophie des Restmyokards nach Herzinfarkt, ischämische Kardiomyopathie, Hypertrophie des Myokards bei Klappenvitien und Myokarditis bei toxischen oder i-atrogenen Einflüssen positiv beeinflussen können.

Überraschenderweise wurde nun gefunden, dass Zatebradine, Cilobradine oder Alinidine insbesondere jedoch Cilobradine bevorzugt sind, nicht nur die klinische Symptomatik einer hypertrophischen Cardiomyopathie günstig zu beeinflussen, sondern sogar eine Regression dieser schweren Herzerkrankungen, zu induzieren.

Gegenstand der vorliegenden Erfindung ist somit die neue Verwendung von Zatebradine, Cilobradine oder Alinidine insbesondere jedoch Cilobradine zur Induktion der Regression von mit Hypertrophie einhergehenden Myocarderkrankungen, insbesondere zur Behandlung von idiopathischen hypertrophischen Kardiomyopathien (HCM) bei Mensch und Haustier.

Zur Erzielung der erfindungsgemäßen Wirkung wird zweckmäßigerweise die zur Behandlung der erhöhten Herzfrequenz aus der Literatur für die einzelnen bradykarden Substanzen bekannte Dosierung verwendet. Beispielsweise beträgt die Einzeldosis
für Cilobradine 0.1 bis 0.5 mg/kg per os, vorzugsweise 0.2 bis 0.4 mg/kg, 1 bis 3 x täglich,
für Zatebradine 0.2 bis 1 mg/kg 2 x täglich sowie
für Alinidine 0.5 bis 5 mg/kg 2 x täglich.

Die erfindungsgemäße neue Verwendung der bradykarden Substanzen wurde am Beispiel des i_{f}-Kanalblockers Cilobradine wie folgt geprüft:

Eine Katze mit schwerer hypertrophischer Kardiomyopathie (Herzfrequenzen ca. 200 Schläge/Minuten), EKG mit ST-Anhebungen als Zeichen myokardialer Ischämie, erhöhter Kreatininkinaseaktivität im Plasma und im Ultraschallbild, massive Verdichtung der Ventrikelwand bei Reduktion des Ventrikelvolumens und der Auswurffraktion, zeigte nach Behandlung mit dem i_{f}-Kanalblocker Cilobradine (2 x täglich 0.3 mg/kg per os) eine deutliche Verbesserung der klinischen Symptomatik (Nachlassen der Schmerzhaftigkeit, EKG-Normalisierung, Wiederaufnahme des normalen physiologischen Aktivitätsmusters).

Bei Nachuntersuchungen nach einem Jahr und nach ca. 2 Jahren Behandlungsdauer zeigt sich überraschenderweise eine Regression der Myokardhypertrophie bei weiterhin erhaltener Verbesserung der Symptomatik.

Die hypertrophische Kardiomyopathie der Katze gilt als Modell für die entsprechende Erkrankung der Menschen (Kittleson et al., Circulation 91, 3172-3180 (1999)).

Die Behandlung mit dem i_{f}-Kanalblocker Cilobradine führt somit neben einer Verbesserung der Symptomatik zu einer Regression der Erkrankung.

Ein weiterer Gegenstand der vorliegenden Erfindung sind Arzneimittelkombinationen, enthaltend mindestens eine bradykarde Substanz, insbesondere eine der vorstehend erwähnten Verbindungen, wobei ein i_{f}-Kanalblocker bevorzugt ist, und mindestens eine herzwirksame Substanz wie
ein Herzglycosid, z.B. Methyldigoxin oder Digitoxin,
einen Vasodilatator, z.B. Nitroglycerin,
einen ACE-Hemmer, z.B. Captopril oder Enalapril,
einen Angiotensin-II-Antagonisten, z.B. Losartan oder Telmisartan,
welche ebenfalls zur Behandlung von mit Hypertrophie einhergehenden Myocarderkrankungen, insbesondere zur Behandlung von idiopathischen hypertrophischen Kardiomyopathien (HCM) geeignet sind, wenn durch eine Kombination mit einer bradykarden Substanz ein Anstieg der Herzfrequenz verhindert werden kann.

Zur Erzielung der erfindungsgemäßen Wirkung wird zweckmäßigerweise die zur Behandlung der erhöhten Herzfrequenz aus der Literatur für die einzelrien bradykarden Substanzen sowie die der für die eingesetzte herzwirksame Verbindung aus der Literatur bekannten Dosierungen verwendet.

Hierzu werden die bradykarden Substanzen entweder alleine oder in Kombination mit anderen herzwirksamen Verbindungen mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/-Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet.

So enthält beispielsweise die Kombination bestehend aus Cilobradine und einer herzwirksamen Verbindung, zweckmäßigerweise. 0.1 bis 0.5 mg/kg, vorzugsweise 0.2 bis 0.4 mg/kg Cilobradine per os
plus 0.01 bis 1 mg Methyldigoxin, 1 bis 2 x täglich,
0.01 bis 1 mg Digoxin, 1 x täglich,
0.1' bis 2 mg Nitroglycerin, 2 bis 3 x täglich,
10 bis 100 mg Captopril, 1 bis 2 x täglich,
2 bis 20 mg Enalapril, 1 x täglich,
10 bis 200 mg Losartan, 2 x täglich, oder
20 bis 80 mg Telmisartan, 1 x täglich.

Da die Kombinationspartner für die i_{f}-Kanalblocker zusätzlich an unabhängigen biologischen System angreifen und i_{f}-Kanalblocker reflektorische Herzfrequenzerhöungen hemmen, welche im Zusammenhang mit den obigen Kombinationspartner auftreten können, weisen diese eine synergistische Wirkungsweise auf.

Die nachfolgenden Beispiele sollen die Erfindung näher erläutern, ohne diese zu beschränken:

### Beispiel 1

### Kapseln zu 1,25 mg Cilobradine

### Zusammensetzung:

### 1 Kapsel enthält:

| | |
|---|---|
| Lactosemonohydrat | 82,75 mg |
| Maisstärke | 55,3 mg |

### Herstellungsverfahren

Der Wirkstoff, Lactosemonohydrat und Maisstärke werden gemischt und in Kapseln Größe 4 abgefüllt.

### Beispiel 2

### Kapseln zu 10 mg Cilobradine

### Zusammensetzung:

### 1 Kapsel enthält:

| | |
|---|---|
| Lactosemonohydrat | 77,6 mg |
| Maisstärke | 51,7 mg |

### Herstellungsverfahren

Der Wirkstoff, Lactosemonohydrat und Maisstärke werden gemischt und in Kapseln Größe 4 abgefüllt.

### Beispiel 3

### Tabletten zu 7,5 mg Cilobradine

### Zusammensetzung:

### 1 Tablette enthält:

| | |
|---|---|
| Wirksubstanz | 7,5 mg |
| Maisstärke | 59,5 mg |
| Milchzucker | 48,0 mg |
| Polyvinylpyrrolidon | 4,0 mg |
| Magnesiumstearat | 1,0 mg |
| | 120,0 mg |

### Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei 45°C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 120 mg

### Beispiel 4

### Dragées zu 5 mg Cilobradine

### 1 Drageekern enthält:

| | |
|---|---|
| Wirksubstanz | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

### Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Drageekerne mit einem Durchmesser von 6 mm gepreßt. Die so hergestellten Drageekerne werden auf bekannte Weise mit einer Schicht überzogen, die im wesentlichen aus Zucker und Talkum besteht. Die fertigen Dragées werden mit Wachs poliert.
Dragéegewicht: 130 mg

### Beispiel 5

### Ampullen zu 5 mg Cilobradine

### 1 Ampulle enthält:

| | |
|---|---|
| Wirksubstanz | 5,0 mg |
| Sorbit | 50,0 mg |
| Wasser für Injektionszwecke ad | 2,0 mg |

### Herstellungsverfahren

In einem geeigneten Ansatzgefäß wird der Wirkstoff in Wasser für Injektionszwecke gelöst und die Lösung mit Sorbit isotonisch gestellt.

Nach Filtration über einem Membranfilter wird die Lösung unter N₂-Begasung in gereinigte und sterilisierte Ampullen abgefüllt und 20 Minuten im, strömenden Wasserdampf autoklaviert.

### Beispiel 6

### Suppositorien zu 10 mg Cilobradine

### 1 Zäpfchen enthält:

| | |
|---|---|
| Wirksubstanz | 0,010 g |
| Hartfett (z.B. Witepsol H 19 und W 45) | 1,690 g |
| | 1,700 g |

### Herstellungsverfahren

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

### Beispiel 7

### Tropfenlösung mit 10 mg Cilobradine

### 100 ml Lösung enthalten:

| | |
|---|---|
| Wirksubstanz | 0,2 g |
| Hydroxyethylcellulose | 0,15 g |
| Weinsäure | 0,1 g |
| Sorbitlösung 70 % Trockensubstanz | 30,0 g |
| Glycerin | 10,0 g |
| Benzoesäure | 0,15 g |
| Dest. Wasser ad | 100 ml |

### Herstellungsverfahren

Dest. Wasser wird auf 70°C erhitzt. Hierin wird unter Rühren Hydroxyethylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

## Patentansprüche

1. Verwendung von Zatebradine, Cilobradine oder Alinidine, und gegebenenfalls eine herzwirksame Verbindung, zur Herstellung eines Arzneimittels zur Behandlung von mit Hypertrophie einhergehenden Myocarderkrankungen.

2. Verwendung gemäß Anspruch 1, **dadurch gekennzeichnet, dass** Zatebradine verwendet wird.

3. Verwendung gemäß Anspruch 1, **dadurch gekennzeichnet, dass** Cilobradine verwendet wird.

4. Verwendung gemäß Anspruch 1, **dadurch gekennzeichnet, dass** Alinidine verwendet wird.

5. Verwendung gemäß den Ansprüchen 1 bis 4, **dadurch gekennzeichnet, dass** als weitere herzwirksame Verbindung ein Herzglycosid; einen Vasodilatator, einen ACE-Hemmer oder einen Angiotensin-II-Antagonisten verwendet wird.

## Claims

1. Use of zatebradine, cilobradine or alinidine, and optionally a cardioactive compound, for preparing a medicament for the treatment of myocardial diseases accompanied by hypertrophy.

2. Use according to claim 1, **characterised in that** zatebradine is used.

3. Use according to claim 1, **characterised in that** cilobradine is used.

4. Use according to claim 1, **characterised in that** alinidine is used.

5. Use according to claims 1 to 4, **characterised in that** a cardioglycoside, a vasodilator, an ACE-inhibitor or an angiotensin-II antagonist is used as a further cardioactive compound.

## Revendications

1. Utilisation de la zatébradine, de la cilobradine ou de l'alinidine, et éventuellement d'un composé actif sur le coeur, pour la production d'un médicament pour le traitement des maladies myocardiques accompagnées d'une hypertrophie.

2. Utilisation selon la revendication 1 **caractérisée en ce que** la zatébradine est utilisée.

3. Utilisation selon la revendication 1 **caractérisée en ce que** la cilobradine est utilisée.

4. Utilisation selon la revendication 1 **caractérisée en ce que** l'alinidine est utilisée.

5. Utilisation selon les revendications 1 à 4 **caractérisée en ce qu'**un glucoside cardiotonique, un vasodilatateur, un inhibiteur d'ACE ou un antagoniste de l'angiotensine II est utilisé comme autre composé actif sur le coeur.
